# EUROPEAN PATENT APPLICATION

(11) **EP 3 252 007 A1**
(43) Date of publication of application: **06.12.2017**
(21) Application number: 15857135.6
(22) Date of filing: 27.10.2015
(51) Int. Cl.: C01B 33/04, C08G 77/60

(54) **SILANE POLYMERIZATION INHIBITOR**

(30) Priority: 04.11.2014 JP 2014224512
(71) Applicant: Thin Film Electronics ASA, 0230 Oslo (NO)
(72) Inventor: GOTO, Yuichi, Toyama-shi Toyama 939-2753 (JP); ENDO, Masahisa, Toyama-shi Toyama 939-2753 (JP); SON, Gun, Toyama-shi Toyama 939-2753 (JP)
(74) Representative: Awapatent AB
(86) International application number: PCT/JP2015/080244
(87) International publication number: WO 2016/072320

(57) **Abstract**

[Problem] To provide a polymerization inhibitor for silane that, when added at the time of heating distillation, enables purification of silane to a high degree because a polymer is not formed even if heating by distillation is performed and cyclic silane can be present as a monomer. To provide a composition containing polysilane obtained by using a polymerization inhibitor to obtain high-purity cyclic silane, in particular high-purity cyclopentasilane and polymerizing the cyclic silane is applied onto a substrate as a coating-type polysilane composition and fired, and this produces a good silicon thin film with high conductivity.

[Solution] A polymerization inhibitor for silane, the polymerization inhibitor including secondary or tertiary aromatic amines. The silane is cyclic silane. The silane is cyclopentasilane. The aromatic amines are secondary aromatic amines. The aromatic group is a phenyl group or a naphthyl group. The polymerization inhibitor is contained at a proportion of 0.01 to 10 mol% per mole of the silane. In the polymerization inhibitor, a boiling point of the aromatic amines is 196°C or higher.

## Description

### TECHNICAL FIELD

The present invention relates to cyclic silane and a production method thereof. The present invention relates to a silane polymer that is applied to uses in integrated circuits, thin film transistors and the like.

### RELATED ART

Silicon semiconductors have been examined as a material for a thin film transistor (TFT) and solar cells from long ago.

Patterning of a silicon thin film applied to integrated circuits and thin film transistors is generally performed by forming a silicon film by vacuum processes such as CVD. This poses issues that, for example, because such devices employ vacuum processes, they have to be large-scale ones, and also raw materials are gases, which are hard to handle.

As a solution to these issues, there is a technique of applying onto a substrate a silane polymer dissolved in an organic solvent and, after firing, forming a silicon film by dehydrogenation.

A patent document describes a method in which a solution composition containing cyclopentasilane is prepared, this solution composition is irradiated with ultraviolet light, and thereafter this coating film is heated to form a silicon film (please see Patent Document 1).

A patent document describes a silane polymer production method characterized in that it generates a silane polymer having a weight-average molecular weight of 800 to 5000 in terms of polystyrene measured by gel permeation chromatography by irradiating a silane compound having photopolymerizability with a ray of light having a wavelength of 405 nm (please see Patent Document 2).

A patent document discloses a silane composition for semiconductor thin film-formation characterized in that it: contains a polysilane compound in solid form synthesized by irradiating cyclopentasilane with light having a wavelength of 170 to 600 nm, (B) cyclopentasilane and (C) at least one compound selected from a boron compound, an arsenic compound, a phosphorous compound and an antimony compound; is formed by dissolving the polysilane compound in solid form; and has a proportion of the polysilane compound to (B) the cyclopentasilane of 0.1 to 100% by weight (please see Patent Document 3).

A patent document discloses silylcyclopentasilane to be used as a radical initiator for decyclization polymerization of cyclopentasilane (please see Patent Document 4).

A patent document discloses a composition that: consists of hydrogen and silicon and/or germanium and contains oligosilane or polysilane having a molecular weight of 450 to 2300; forms an oligo or polysilane film if the composition is applied and printed; and then, after being cured, forms a noncrystalline hydrogenated semiconductor film having a carbon content equal to or lower than 0.1 atomic % (please see Patent Document 5). It is described that a heterogeneous catalyst formed of Group VII to XII transition metal elements or base material-sticking derivatives thereof is used to synthesize polysilane.

### BACKGROUND DOCUMENTS

### PATENT DOCUMENTS

Patent Document 1: Japanese Patent Application Publication No. 2001-262058
Patent Document 2: Japanese Patent Application Publication No. 2005-22964
Patent Document 3: Japanese Patent Application Publication No. 2003-124486
Patent Document 4: Japanese Patent Application Publication No. 2001-253706
Patent Document 5: Japanese Patent Application Publication No. 2010-506001 (translation)

### SUMMARY OF INVENTION

### PROBLEMS TO BE SOLVED BY INVENTION

If high-purity cyclic silane, in particular high-purity cyclopentasilane, is to be obtained, purification by distillation is performed at the final stage. However, highly reactive cyclic silane such as cyclopentasilane starts polymerization before purification by heating.

An object of the present invention is to provide a polymerization inhibitor to be added at the time of heating distillation in order for cyclic silane to be present as a monomer without forming a polymer even if heating by distillation is performed.

### MEANS FOR SOLVING PROBLEMS

A first aspect of the present invention provides a polymerization inhibitor for silane, the polymerization inhibitor including secondary or tertiary aromatic amines.

A second aspect provides the polymerization inhibitor according to the first aspect, wherein the silane is cyclic silane.

A third aspect provides the polymerization inhibitor according to the first aspect, wherein the silane is cyclopentasilane.

A fourth aspect provides the polymerization inhibitor according to any one of the first aspect to the third aspect, wherein the aromatic amines are secondary aromatic amines.

A fifth aspect provides the polymerization inhibitor according to any one of the first aspect to the third aspect, wherein an aromatic group of the aromatic amines is a phenyl group or a naphthyl group.

A sixth aspect provides the polymerization inhibitor according to any one of the first aspect to the fifth aspect, including a polymerization inhibitor at a proportion of 0.01 to 10 mol% per mole of the silane.

A seventh aspect provides the polymerization inhibitor according to any one of the first aspect to the sixth aspect, wherein a boiling point of the aromatic amines is 196°C or higher.

An eighth aspect provides a silane purification method using a polymerization inhibitor, the method including: Process (A); Process (B); and Process (C), wherein

Process (A) is a process of obtaining a solution containing cyclic silane expressed by Formula (2)
[Chemical Formula 2]

(SiR³R⁴)ₙ Formula (2)

(in Formula (2), R3 and R4 respectively indicate halogen atoms, and n is an integer of 4 to 6) by causing cyclic silane in which a ring is formed by successively present Si and that is expressed by Formula (1):
[Chemical Formula 1]

(SiR¹R²)ₙ Formula (1)

(in Formula (1), R1 and R2 respectively indicate a hydrogen atom, C1 to C6 alkyl groups or optionally substituted phenyl groups (R1 and R2 are not hydrogen atoms simultaneously), and n is an integer of 4 to 6) to react with a hydrogen halide in cyclohexane in the presence of an aluminum halide,

Process (B) is a process of obtaining cyclic silane expressed by Formula (3):
[Chemical Formula 3]

(SiH₂)n Formula (3)

(in Formula (3), n is an integer of 4 to 6) by dissolving the cyclic silane expressed by Formula (2) in an organic solvent and reducing the cyclic silane expressed by Formula (2) with hydrogen or a lithium aluminum hydride, and

Process (C) is a process of generating cyclic silane expressed by Formula (3) by adding the polymerization inhibitor according to any one of the first aspect to the seventh aspect to the cyclic silane expressed by Formula (3) and further distilling a resultant matter.

A ninth aspect provides the silane purification method according to the eighth aspect, wherein after obtaining the solution containing the cyclic silane expressed by Formula (2), Process (A) includes a process of generating the cyclic silane expressed by Formula (2) by further distilling the solution.

A tenth aspect provides a silane preservation method including adding the polymerization inhibitor according to any one of the first aspect to the seventh aspect to a silane-containing organic solvent.

### EFFECTS OF INVENTION

The present invention provides a useful polymerization inhibitor to be added at the time of heating distillation in a distillation process performed at the final stage to obtain high-purity cyclic silane, in particular high-purity cyclopentasilane. That is, due to addition of the polymerization inhibitor according to the present invention, cyclic silane can be allowed to be present as a monomer without forming a polymer of silane even if heating distillation is performed.

Also, in the present invention, using a polymerization inhibitor containing secondary or tertiary aromatic amines as a polymerization inhibitor to be used at the time of distillation of highly reactive silane, unnecessary polymerization of silane can be suppressed, and silane purified as a stable monomer can be obtained.

Furthermore, also, in the present invention, the polymerization inhibitor can be utilized as a polymerization inhibitor at the time of preservation. That is, by adding the polymerization inhibitor in a solution of the silane monomer in an organic solvent, polymerization of the silane monomer can be suppressed, and it can be preserved stably.

### EMBODIMENTS FOR IMPLEMENTING INVENTION

The present invention relates to a polymerization inhibitor for silane, the polymerization inhibitor including secondary or tertiary aromatic amines. Silane utilized may be straight-chain silane, branched silane, cyclic silane, or a mixture of them. In particular, highly reactive cyclic silane is preferable, and examples of the number of cyclic silane include 4 to 6. For example, examples thereof include cyclotetrasilane, cyclopentasilane and cyclohexasilane, and among them, cyclopentasilane is suitably used.

Among secondary and tertiary aromatic amines, secondary aromatic amines are preferable because they provide higher polymerization inhibiting effects.

Secondary aromatic amines are formed by two aromatic groups and one hydrogen atom being directly bonded to a nitrogen atom, and tertiary aromatic amines are formed by three aromatic groups being directly bonded to a nitrogen atom.

Aromatic groups have the number of carbon atoms of 6 to 40. For example, examples thereof include a phenyl group, a naphthyl group, an anthryl group, a biphenyl group or the like, and in particular, a phenyl group and a naphthyl group are preferably used.

An example of the polymerization inhibitor used in the present invention can be expressed as:

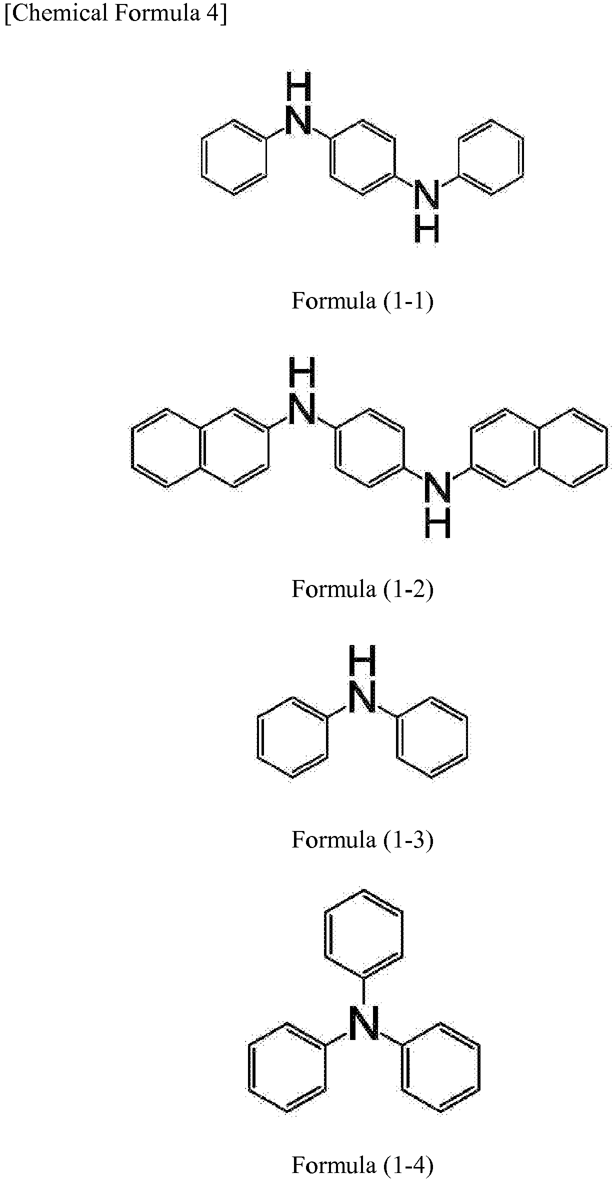

The boiling point of N,N'-diphenyl-1,4-diphenylenediamine in Formula (1-1) is 220 to 225°C, the boiling point of N,N'-di-2-naphthyl-1,4-diphenylenediamine in Formula (1-2) is 608°C, the boiling point of diphenylamine in Formula (1-3) is 302°C, and the boiling point of triphenylamine in Formula (1-4) is 365°C.

The polymerization inhibitor added may be contained at a proportion of 0.01 to 10 mol%, 0.01 to 5 mol%, or 0.01 to 1 mol% per mole of silane. If silane is a mixture of various types of silane, assuming that the entire mass of silane is constituted by cyclopentasilane, the polymerization inhibitor may be added at the above-mentioned proportion in terms of the number of moles of cyclopentasilane.

Also, the cyclic silane expressed by Formula (1) used as a raw material at the time of synthesizing cyclopentasilane of interest may be a commercial product. If the synthesis is performed, silane expressed by Formula (a):
[Chemical Formula 5]

R¹R²SiX₂ Formula (a)

(where in Formula (a), R¹ and R² respectively indicate hydrogen atoms, C₁ to C₆ alkyl groups, or optionally substituted phenyl groups, and X indicates a halogen atom) may be caused to undergo a reaction in the organic solvent in the presence of an alkali metal; thereby, the cyclic silane expressed by Formula (1) may be obtained.

Here, examples of the C₁ to C₆ alkyl group include a methyl group, an ethyl group, an n-propyl group, an i-propyl group, a cyclopropyl group, an n-butyl group, an i-butyl group, an s-butyl group, a t-butyl group, a cyclobutyl group, a 1-methyl-cyclopropyl group, a 2-methyl cyclopropyl group, an n-pentyl group, etc. Examples of the substituent in the optionally substituted phenyl group include the alkyl group. Examples of the halogen atoms include fluorine atoms, chlorine atoms, bromine atoms and iodine atoms, and chlorine atoms may be used preferably. The alkali metal is lithium, sodium, potassium or the like. If the alkali metal is dispersed in an organic solvent such as tetrahydrofuran, and furthermore the silane expressed by Formula (a) is added thereto, the cyclic silane expressed by Formula (1) is generated. The amount of the alkali metal used at this time is, in the unit of mole, approximately 1.5 to 3 times that of the silane expressed by Formula (a). This reaction occurs at room temperature, and an obtained product is purified by recrystallization or the like.

Examples of silane expressed by Formula (a) include diphenyldichlorosilane, diphenyldibromosilane, diphenyldiiodosilane, di(phenyl chloride)dichlorosilane, dimethyldichlorosilane, dimethyldibromosilane, etc.

Also, the present invention relates to a silane purification method using the above-mentioned polymerization inhibitor. The silane purification method includes for example Process (A), Process (B) and Process (C).

Examples of a substituent on C₁ to C₆ alkyl groups and optionally substituted phenyl groups in the cyclic silane expressed by Formula (1) used in Process (A) include the above-mentioned examples. In Formula (1), n is an integer of 4 to 6. Preferably, only cyclic silane in which n = 5 is used or alternatively cyclic silane containing cyclic silane in which n = 5 as a major component may be used. Cyclic silane in which n = 5 and in which R¹ and R² are phenyl groups is decaphenylcyclopentasilane, which may be preferably used as a raw material. Cyclic silane in which n = 4 or n = 6 may also be contained in the cyclic silane.

Process (A) is a process of obtaining a solution containing cyclic silane expressed by Formula (2) by causing cyclic silane in which a ring is formed by successively present Si and that is expressed by Formula (1) to react with halogen or a hydrogen halide. Also, after obtaining the solution containing cyclic silane expressed by Formula (2), it may further include a process of generating cyclic silane expressed by Formula (2) by distillation. At Process (A), distillation is performed at a temperature of 40 to 80°C and a degree of pressure reduction of 0 to 30 Torr (for example, 1 to 30 Torr, or 5 to 30 Torr) for 2 to 24 hours.

At that time, a reaction may be cause to occur in an organic solvent (for example, cyclohexane, hexane, heptane, toluene benzene) with an aluminum halide (for example, aluminum chloride, aluminum bromide) as a catalyst. The amount of the hydrogen halide (for example, hydrogen chloride) required is 2n moles or more relative to the cyclic silane, and for example may be 2.5n moles to 3.5n moles, and an excess amount of it may be added. The catalyst may be added at a proportion of 0.01 moles to 2 moles per mole of cyclic silane. If hydrogen chloride is used, at Process (A), cyclic silane expressed by Formula (2) (R³ and R⁴ in the formula are chlorine atoms) can be obtained.

Process (B) is a process of obtaining the cyclic silane expressed by Formula (3) by reducing the cyclic silane expressed by Formula (2) with hydrogen or a lithium aluminum hydride.

Specifically, Process (B) is a process of dissolving the compound expressed by Formula (2) in the organic solvent (for example, cyclohexane, hexane, heptane, toluene, benzene), slowly adding a lithium aluminum hydride dissolved in ether (for example, diethyl ether, tetrahydrofuran, cyclopentimethyl ether) thereto, reducing the cyclic silane expressed by Formula (2), and converting the cyclic silane expressed by Formula (2) into the cyclic silane expressed by Formula (3). The lithium aluminum hydride added at this time may be added at a proportion of 2 to 3 moles per mole of the cyclic silane expressed by Formula (2).

In Formula (3), n is an integer of 4 to 6. Cyclopentasilane in which n is 5 is preferably contained at a proportion of no less than 80 mol%, for example 80 to 100 mol% and 90 to 100 mol% in the entire silane to be obtained. In particular, cyclopentasilane preferably of high-purity (100 mol%) is preferably obtained.

Process (C) is a process of adding a polymerization inhibitor to the cyclic silane expressed by Formula (3) and further performing distillation to generate the cyclic silane expressed by Formula (3). At Process (C), distillation is performed at a temperature of 20 to 70°C and at a degree of pressure reduction of 1 to 50 Torr (for example, 1 to 35 Torr, or 2 to 50 Torr) for 4 to 6 hours.

In the present invention, the polymerization inhibitor is used at the time of distillation of silane at the final stage. As its method, the polymerization inhibitor is added to a solvent in which synthesized silane is dissolved, and silane is distilled.

In this method, distillation is performed to increase the purity of silane, and as a polymerization inhibitor to be added at that time, one that has a boiling point higher than the boiling point of silane is preferable. In particular, in order for cyclopentasilane to be used suitably as silane, the boiling point of the polymerization inhibitor may be higher than the boiling point of cyclopentasilane (195°C), for example the boiling point may be 196°C or higher or preferably 200°C or higher, and aromatic amines for example having a boiling point in a range no greater than 700°C may be used.

Conventionally, generally a monomer of cyclic silane, in particular cyclopentasilane, is required to have high-purity quality. In such a case, conventionally, high-purity silane is polymerized and applied onto a substrate as polysilane, and a uniform coating film is formed. In the silane monomer in this coating film, Si-H bonds are broken by suitable heating, and polysilane having Si-Si bonds is generated.

Such a silane monomer is preferably present as a monomer that can undergo distillation or the like until it is purified to a high degree. In view of this, in synthesis of cyclic silane, in particular cyclopentasilane, distillation is performed at the final stage of synthesis for a high degree of purification. This distillation is performed at reduced pressure and raised temperature, but cyclic silane such as cyclopentasilane is highly reactive, and may undergo polymerization at the time of distillation.

The present invention provides a method that suppresses unnecessary polymerization and makes it possible to obtain a stable silane monomer using secondary or tertiary aromatic amines as a polymerization inhibitor to be used at the time of distillation of highly reactive silane.

Thus, in the present invention, a polymer of polysilane, for example cyclopentasilane, obtained by polymerizing purified high-purity cyclic silane, for example cyclopentasilane may be obtained. These types of polysilane may be applied onto a substrate to form a silicon coating film. The polymerization may be performed by a method using a catalyst or a method using thermal polymerization.

The obtained polysilane is a polymer of cyclopentasilane, for example, and it is obtained as a solution in the organic solvent of 1% by mass to 20% by mass. For example, a transparent solution is obtained even if the organic solvent (cyclohexane) of 13.5% by mass is obtained.

The obtained polymer of the cyclopentasilane has a weight-average molecular weight of approximately 600 to 3000, and this gives the Mw/Mn ratio between the weight-average molecular weight Mw and the number-average molecular weight Mn of 1.03 to 1.55; thus, a polymer with a narrow molecular weight distribution is obtained.

The polymer can be obtained at a high yield in the range of 80 to 90%.

Furthermore, the present invention relates to a silane preservation method, that is, a method of preserving silane by adding the above-mentioned polymerization inhibitor to an organic solvent containing silane. Utilizing it as a polymerization inhibitor at the time of preservation, and adding the polymerization inhibitor into a solution of an organic solvent containing the silane monomer allow suppression of polymerization of the silane monomer.

Also, the obtained polysilane product is obtained by removing volatile components by reducing pressure, and can be preserved dissolved in a solvent. Examples of the solvent for the polysilane include: a hydrocarbon-based solvent such as n-hexane, n-heptane, n-octane, n-decane, cyclohexane, cyclooctane, dicyclopentane, benzene, toluene, xylene, duren, indene, tetrahydronaphthalene; decahydronaphthalene or squalane; an ether-based solvent such as dipropyl ether, ethylene glycol dimethyl ether, ethylene glycol diethyl ether, ethylene glycol methyl ethyl ether, diethylene glycol dimethyl ether, diethylene glycol diethyl ether, diethylene glycol methyl ethyl ether, tetrahydrofuran, tetrahydropyran, 1,2-Dimethoxyethane, bis(2-methoxyethyl)ether, or p-dioxane; and furthermore propylene carbonate, γ-butyrolactone, N-methyl-2-pyrrolidone, dimethyl formamide, acetonitrile, dimethyl sulphoxide, etc. Among the above-mentioned examples of the solvent, cyclooctane is preferably used, and a polysilane composition can be formed by causing cyclooctane to contain the polysilane in the amount of 5 to 8 % by mass.

A substance including Group IIIB elements and Group VB elements may be added to polysilane as a dopant. Examples of those substances include compounds such as phosphorous or boron. Apolysilane composition to which such a dopant is added may be applied onto a base material to form an n-type or p-type silicon film after performing a process such as heating.

In a method of forming a silicon film, the polysilane composition is applied onto a substrate, and heat treatment or the like is performed to obtain a silicon film by dehydrogenation. The coating is performed using a device for spin coating, roll coat, dip coat or the like, and after the coating, heat treatment is performed. For example, spin coating is performed with the number of rotation of a spinner being set to 500 to 1000 rpm.

The coating process is preferably performed in inert gas atmosphere, and is for example performed while a nitrogen gas, a helium gas, an argon gas or another gas is being allowed to flow.

The coated substrate undergoes heat treatment, the heating temperature is 100 to 425°C, and the process lasts for 10 to 20 minutes.

The film thickness of the thus-obtained silicon film is in a range of 60 to 100 nm.

Examples of the substrate include: a transparent electrode such as quartz, glass or ITO; a metal electrode such as gold, silver, copper, nickel, titanium, aluminum or tungsten; a glass substrate; a plastic substrate; etc.

### EXAMPLES

The weight-average molecular weight can be measured by gel permeation chromatography (GPC) (measurement equipment: HLC-8320GPC (manufactured by Tosoh Corporation); column: GPC/SEC (PLgel 3 µm, 300 x 7.5 mm, manufactured by Varian Inc.); column temperature: 35°C; detector: RI; flow rate: 1.0 ml/min; measurement time: 15 minutes; eluent: cyclohexane; injection amount: 10 µL); sample concentration: 1.0% (in cyclohexane). Also, with CPS (Mw150, RT = 11.040 minutes), CPS-dimer (Mw298, RT = 10.525 minutes) and CPS-trimer (Mw446, RT = 9.725 minutes) as primary standards, a calibration curve was generated. The polymerization progress indicating the degree of progress of polymerization is defined as: the ratio of the area of the spectrum indicated by initial CPS occupying the entire spectrum - the ratio of the area of the spectrum indicated by CPS after 4 hours occupying the entire spectrum)/the ratio of the area of the spectrum indicated by initial CPS occupying the entire spectrum) x 100.
CPS means cyclopentasilane.

### [Synthesis Example 1] Synthesis of Decachlorocyclopentasilane

In nitrogen atmosphere, decaphenylcyclopentasilane (500.0 g) and cyclohexane (453.7 g) were put into a 2L-reaction flask as a solvent. After aluminum chloride AlCl₃ (14.7 g) was added to it, the temperature of the resultant mixture was raised to room temperature in a water bath. A hydrogen chloride HCL gas was blown on it at a flow velocity (280 mL/min) for 8 hours. Thereafter, after pressure reduction and pressure recovery by means of nitrogen were repeated ten times to remove hydrogen chloride, the resultant mixture was filtered using a membrane filter; thereby, a cyclohexane solution of decachlorocyclopentasilane (1099.5 g) was obtained.

### [Synthesis Example 2] Synthesis of Cyclopentasilane

Solvent removal was performed at 20 to 30°C and 25 Torr for 2 hours on the cyclohexane solution of decachlorocyclopentasilane (1099.5 g) obtained in Synthesis Example 1, and thereafter the resultant mixture was distilled at 60°C and 13 Torr for 4 hours; thereby, decachlorocyclopentasilane (268.56 g) from which cyclohexylbenzene was removed was obtained. After cyclohexane (814.5 g) was added to and dissolved in it, the resultant fixture was filtered using a membrane filter, and rinsed using cyclohexane (50 g); thereby, a cyclohexane solution of high-purity decachlorocyclopentasilane (1100.6 g) was obtained. This was put into a 2L-reaction flask in argon atmosphere, and at 0 to 10°C, a diethyl ether (269.6 g) solution of lithium aluminum hydride LiAlH₄ (57.5 g) was dripped over 2 hours. After the resultant mixture was agitated at room temperature for 1 hour, at 0 to 10°C, ion-exchanged water (592.7 g) was dripped onto the reaction solution over 1 hour. After being agitated for 10 minutes and allowed to stand still, water layer parts were removed. Subsequently, ion-exchanged water (592.7 g) was added to it at room temperature, and this rinsing operation was performed four times. Thereafter, the organic layer was dried for 1 hour using magnesium sulfate (23.7 g), and then filtration using a membrane filter and concentration were performed to obtain a cyclopentasilane (71.8 g).

### [Example 1] Addition of DPPA (N,N'-Diphenyl-1,4-Diphenylenediamine)

In argon atmosphere, cyclopentasilane (3.0 g) obtained in Synthesis Example 2 was put into a 30-mL-reaction flask in the presence of, as a polymerization inhibitor, DPPA (N,N'-diphenyl-1,4-diphenylenediamine) (0.055 g, 1.0 mol%), and heating was performed thereon at 70°C for 4 hours. Analysis by gel permeation chromatography (GPC) performed showed that the polymerization progress was less than 1%.

### [Example 2] Addition of DNPA

### (N,N'-Di-2-Naphthyl-1,4-Diphenylenediamine)

In argon atmosphere, cyclopentasilane (3.0 g) obtained in Synthesis Example 2 was put into a 30-mL-reaction flask in the presence of, as a polymerization inhibitor, DNPA (N,N'-di-2-naphthyl-1,4-diphenylenediamine) (0.075 g, 1.0 mol%), and heating was performed thereon at 70°C for 4 hours. Analysis by gel permeation chromatography (GPC) performed showed that the polymerization progress was less than 1%.

### [Example 3] Addition of DPA (Diphenylamine)

In argon atmosphere, cyclopentasilane (3.0 g) obtained in Synthesis Example 2 was put into a 30-mL-reaction flask in the presence of, as a polymerization inhibitor, DPA (diphenylamine) (0.034 g, 1.0 mol%), and heating was performed thereon at 70°C for 4 hours. Analysis by gel permeation chromatography (GPC) performed showed that the polymerization progress was 1.3%.

### [Example 4] Addition of TPA (Triphenylamine)

In argon atmosphere, cyclopentasilane (3.0 g) obtained in Synthesis Example 2 was put into a 30-mL-reaction flask in the presence of, as a polymerization inhibitor, TPA (triphenylamine) (0.050 g, 1.0 mol%), and heating was performed thereon at 70°C for 4 hours. Analysis by gel permeation chromatography (GPC) performed showed that the polymerization progress was 7.7 %.

### [Example 5] Addition of DNPA

### (N,N'-Di-2-Naphthyl-1,4-Diphenylenediamine)

In argon atmosphere, cyclopentasilane (5.0 g) obtained in Synthesis Example 2 was put into a 30-mL-reaction flask in the presence of, as a polymerization inhibitor, DNPA (N,N'-di-2-naphthyl-1,4-diphenylenediamine) (0.0125 g, 0.1 mol%), and heating was performed thereon at 70°C for 4 hours. Analysis by gel permeation chromatography (GPC) performed showed that the polymerization progress was less than 1%.

### [Example 6] Addition of DNPA

### (N,N'-Di-2-Naphthyl-1,4-Diphenylenediamine)

In argon atmosphere, cyclopentasilane (5.0 g) obtained in Synthesis Example 2 was put into a 30-mL-reaction flask in the presence of, as a polymerization inhibitor, DNPA (N,N'-di-2-naphthyl-1,4-diphenylenediamine) (0.0013 g, 0.01 mol%), and heating was performed thereon at 70°C for 4 hours. Analysis by gel permeation chromatography (GPC) performed showed that the polymerization progress was 2%.

### [Comparative Example 1] No Polymerization Inhibitor

In argon atmosphere, cyclopentasilane (3.0 g) obtained in Synthesis Example 2 was put into a 30-mL-reaction flask in the absence of polymerization inhibitors, and heating was performed thereon at 70°C for 4 hours. Analysis by gel permeation chromatography (GPC) performed showed that the polymerization progress was 29.1 %.

### [Comparative Example 2] Addition of AN (Aniline)

In argon atmosphere, cyclopentasilane (3.0 g) obtained in Synthesis Example 2 was put into a 30-mL-reaction flask in the presence of, as a polymerization inhibitor, AN (aniline) (0.019 g, 1.0 mol%), and heating was performed thereon at 70°C for 4 hours. Analysis by gel permeation chromatography (GPC) performed showed that the polymerization progress was 26.8%.

### [Comparative Example 3] Addition of DCA (Dicyclohexylamine)

In argon atmosphere, cyclopentasilane (3.0 g) obtained in Synthesis Example 2 was put into a 30-mL-reaction flask in the presence of, as a polymerization inhibitor, DCA (dicyclohexylamine) (1.0 mol%) (0.037 g), and heating was performed thereon at 70°C for 4 hours. Analysis by gel permeation chromatography (GPC) performed showed that the polymerization progress was 21.0%.

### [Comparative Example 4] Addition of HQ (Hydroquinone)

In argon atmosphere, cyclopentasilane (3.0 g) obtained in Synthesis Example 2 was put into a 30-mL-reaction flask in the presence of, as a polymerization inhibitor, HQ (hydroquinone) (0.022 g, 1.0 mol%), and heating was performed thereon at 70°C for 4 hours. Analysis by gel permeation chromatography (GPC) performed showed that the polymerization progress was 20.0%.

In the present invention, as shown in the above-mentioned examples, in a silane preservation method or purification method using a polymerization inhibitor, the silane polymerization progress is suitably 15% or lower and preferably 10% or lower.

### INDUSTRIAL APPLICABILITY

A composition containing polysilane obtained by using a polymerization inhibitor to obtain high-purity cyclic silane, in particular high-purity cyclopentasilane and polymerizing the cyclic silane is applied onto a substrate as a coating-type polysilane composition and fired, and this produces a good silicon thin film with high conductivity.

## Claims

1. A polymerization inhibitor for silane, the polymerization inhibitor comprising secondary or tertiary aromatic amines.

2. The polymerization inhibitor according to Claim 1, wherein the silane is cyclic silane.

3. The polymerization inhibitor according to Claim 1, wherein the silane is cyclopentasilane.

4. The polymerization inhibitor according to any one of Claims 1 to 3, wherein the aromatic amines are secondary aromatic amines.

5. The polymerization inhibitor according to any one of Claims 1 to 3, wherein an aromatic group of the aromatic amines is a phenyl group or a naphthyl group.

6. The polymerization inhibitor according to any one of Claims 1 to 5, wherein the polymerization inhibitor is contained at a proportion of 0.01 to 10 mol% per mole of the silane.

7. The polymerization inhibitor according to any one of Claims 1 to 6, wherein a boiling point of the aromatic amines is 196°C or higher.

8. A silane purification method using a polymerization inhibitor, the method comprising: Process (A); Process (B); and Process (C), wherein
Process (A) is a process of obtaining a solution containing cyclic silane expressed by Formula (2)
(SiR³R⁴)ₙ Formula (2)
(in Formula (2), R³ and R⁴ respectively indicate halogen atoms, and n is an integer of 4 to 6) by causing cyclic silane in which a ring is formed by successively present Si and that is expressed by Formula (1):
(SiR¹R²)ₙ Formula (1)
(in Formula (1), R¹ and R² respectively indicate independently a hydrogen atom, C₁ to C₆ alkyl groups or optionally substituted phenyl groups (R¹ and R² are not hydrogen atoms simultaneously), and n is an integer of 4 to 6) to react with a hydrogen halide in cyclohexane in the presence of an aluminum halide,
Process (B) is a process of obtaining cyclic silane expressed by Formula (3):
(SiH₂)n Formula (3)
(in Formula (3), n is an integer of 4 to 6) by dissolving the cyclic silane expressed by Formula (2) in an organic solvent and reducing the cyclic silane expressed by Formula (2) with hydrogen or a lithium aluminum hydride, and
Process (C) is a process of generating cyclic silane expressed by Formula (3) by adding the polymerization inhibitor according to any one of Claims 1 to 7 to the cyclic silane expressed by Formula (3) and further distilling a resultant matter.

9. The silane purification method according to Claim 8, wherein after obtaining the solution containing the cyclic silane expressed by Formula (2), Process (A) includes a process of generating the cyclic silane expressed by Formula (2) by further distilling the solution.

10. A silane preservation method comprising adding the polymerization inhibitor according to any one of Claims 1 to 7 to a silane-containing organic solvent.
